# FASCICULE DE BREVET EUROPEEN

(11) **EP 1 120 104 B1**
(45) Date de publication et mention de la délivrance du brevet: **06.10.2004**
(21) Numéro de dépôt: 00403530.9
(22) Date de dépôt: 14.12.2000
(51) Int. Cl.: A61K 7/06

(54) **Compositions cosmétiques détergentes contenant un amidon amphotère particulier et leurs utilisations**
Kosmetische Reinigungszusammensetzungen, die eine besondere amphotere Stärke enthalten, und deren Verwendung
Cosmetic detergent compositions containing a chosen amphoteric starch and the uses thereof

(30) Priorité: 13.01.2000 FR 0000411
(43) Date de publication de la demande: 01.08.2001
(73) Titulaire: L'OREAL, 75008 Paris (FR)
(72) Inventeur: Maubru, Mireille, 78400 Chatou (FR); Beauquey, Bernard, 92110 Clichy (FR); Douin, Véronique, 75017 Paris (FR)
(74) Mandataire: Le Blainvaux Bellegarde, Françoise

(56) Documents cités:
- EP-A- 0 689 829
- WO-A-00/33806
- WO-A-01/39721
- DE-A- 19 816 665
- DATABASE CHEMICAL ABSTRACTS [en ligne] STN; abstract 119: 167 475, XP002150749 & JP 05 132410 A (KAO CORP.) 28 mai 1993 (1993-05-28)
- DATABASE CHEMICAL ABSTRACTS [en ligne] STN; abstract 119: 167 476, XP002150750 & JP 05 132695 A (KAO CORP.) 28 mai 1993 (1993-05-28)

## Description

La présente invention concerne de nouvelles compositions cosmétiques à propriétés améliorées destinées simultanément au nettoyage et au conditionnement des matières kératiniques telles que les cheveux, et comprenant, dans un support aqueux cosmétiquement acceptable, une base lavante constituée de tensioactifs à pouvoir détergent et un amidon amphotère particulier. L'invention concerne aussi l'utilisation desdites compositions dans l'application cosmétique susmentionnée.

Pour le nettoyage et/ou le lavage des matières kératiniques telles que les cheveux, l'utilisation de compositions détergentes (telles que les shampooings) à base essentiellement d'agents tensioactifs classiques de type notamment anionique, non ionique et/ou amphotère, mais plus particulièrement de type anionique, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Il a été préconisé d'utiliser pour la formulation de shampooings des associations d'amidon non-ionique avec des tensio-actifs anioniques. Cependant, ces associations ne conduisent pas à des résultats cosmétiques satisfaisants.

Il a également été préconisé dans EP 689829 l'association d'un amidon amphotère et de savon pour des compositions de mousse à raser mais ces compositions ne sont pas suffisamment détergentes et l'utilisation de savon pour laver les cheveux présente de gros inconvénients comme le ternissement de la fibre.

J05132410 et J05132695 décrivent des compositions contenant des amidons amphotères à groupements phosphobétaïnes. WO0033806 (opposable selon l'article 54(3) CBE) décrit des compositions non détergentes contenant des amidons amphotères à groupement carboxylates.

Ainsi, à la suite d'importantes recherches menées sur la question, il a maintenant été trouvé par la Demanderesse, qu'en utilisant une base lavante et au moins un amidon amphotère particulier, il est possible d'obtenir des compositions détergentes présentant d'excellentes propriétés cosmétiques, en particulier la facilité de coiffage, la légèreté et la souplesse des cheveux traités et ayant de bonnes propriétés d'usage tel qu'un bon pouvoir lavant intrinsèque et un bon pouvoir moussant.

Les compositions conformes à l'invention confèrent aux cheveux, après rinçage, un remarquable effet traitant qui se manifeste notamment par un apport de légèreté, de discipline et de souplesse.

Ainsi, la présente invention a pour objet de nouvelles compositions cosmétiques détergentes et conditionnantes caractérisée par le fait qu'elles comprennent, dans un milieu aqueux cosmétiquement acceptable:
- 4 à 50% en poids par rapport au poids total de la composition d'une base lavante comprenant un ou plusieurs tensioactifs détergents choisis parmi les tensioactifs anioniques, amphotères, non ioniques et leurs mélanges, et
- au moins un amidon amphotère défini ci-dessous, la concentration en sels d'un métal alcalin ou alcalinoterreux ou d'une amine grasse et d'un acide gras en C10-C18 dans la composition étant inférieure ou égale à 1% en poids dans la composition,
à l'exclusion de la composition de shampooing ci-dessous :

| INGREDIENTS | NOM INCI | |
|---|---|---|
| Standapol A | Ammonium lauryl sulfate | 7,50 |
| Standapol EA-3 | Ammonium laureth sulfate | 17,50 |
| Tegobetaine L-7 | Cocamidopropyl betaine | 5,0 |
| Monamid 1113 | Coamide DEA | 3,0 |
| Solance®¹ | Modified Starch | 0,26 |
| Tween 20 | Polysorbate 20 | 1,00 |
| Chlorure de sodium | Sodium Chloride | 0,75 |
| Glydant | DMDM hydantoin | 0,20 |
| Acide citrique | Citric acid | q.s. pH 6 |
| Eau, D.I. | Water | q.s. 100 |
| q.s. signifie quantité suffisante | | |

| | | |
|---|---|---|
| ¹ Amidon modifié amphotère de National Starch décrit dans le brevet US 5,871,756. | | |

L'invention a également pour objet une composition cosmétique détergente et conditionnante, caractérisée par le fait qu'elle comprend, dans un milieu aqueux cosmétiquement acceptable :
- 4 à 50% en poids par rapport au poids total de la composition d'une base lavante comprenant un ou plusieurs tensioactifs détergents choisis parmi les tensioactifs anioniques, amphotères, non ioniques et leurs mélanges,
- au moins un polymère cationique et
- au moins un amidon amphotère défini ci-dessous,
la concentration en sels d'un métal alcalin ou alcalinoterreux ou d'une amine grasse et d'un acide gras en C10-C18 étant inférieure ou égale à 1% en poids dans la composition,

L'invention a également pour objet l'utilisation en cosmétique des compositions ci-dessus pour le nettoyage et /ou le démaquillage et/ou le conditionnement des matières kératiniques telles que les cheveux et la peau. L'invention a encore pour objet une utilisation de la composition selon l'invention comme shampoing des matières kératiniques.

Par savons d'acides gras, on comprend selon l'invention un sel d'un métal alcalin ou alcalinoterreux ou d'une amine grasse et d'un acide gras en C10-C18.
Par exempte de savons d'acides gras, on comprend selon l'invention que la concentration en savons d'acide gras est inférieure ou égale à 1% en poids, de préférence inférieure à 0,1% en poids. Les compositions conformes à l'invention comprennent nécessairement une base lavante, généralement aqueuse.

Le ou les tensioactifs formant la base lavante peuvent être indifféremment choisis, seuls ou en mélanges, au sein des tensioactifs détergents anioniques, amphotères, non ioniques.

Toutefois, selon l'invention, la base lavante comprend de préférence au moins un ou plusieurs tensioactifs anioniques ou des mélanges d'au moins un ou plusieurs tensioactifs anioniques et d'au moins un ou plusieurs tensioactifs amphotères ou d'au moins un ou plusieurs tensioactifs non ioniques.

Ainsi, selon l'invention, la base lavante peut représenter de 4 % à 50 % en poids, de. préférence de 6 % à 35 % en poids, et encore plus préférentiellement de 8 % à 25 % en poids, du poids total de la composition finale.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels d'alcalinoterreux (de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersulfates et leurs mélanges.

### (ii) Tensioactif(s) non ionique(s) :

Les agents tensioactifs non ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL®, tels que décrits dans les brevets US-2 528 378 et US-2 781 354 et de structures :

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R_{2'}-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂. désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA.

Dans les compositions conformes à l'invention, on utilise de préférence des mélanges d'agents tensioactifs et en particulier des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA sous la dénomination commerciale "MIRANOL® C2M. CONC" en solution aqueuse à 38 % de matière active ou sous la dénomination MIRANOL® C32;
- soit un agent tensioactif amphotère de type zwittérionique tel que les alkylbétaïnes en particulier la cocoylbétaïne commercialisée sous la dénomination "DEHYTON® AB 30" en solution aqueuse à 32 % de MA par la société HENKEL ou les alkylamidobétaïnes telles que la TEGOBETAINE® F50 commercialisée par la société GLODSCHMIDT.

Les compositions selon l'invention comprennent nécessairement un amidon amphotère choisi parmi les composés de formules suivantes : 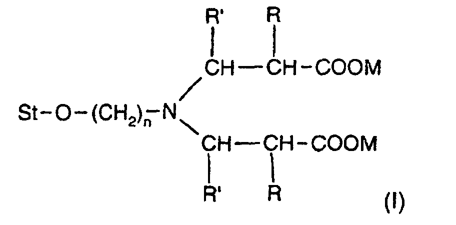 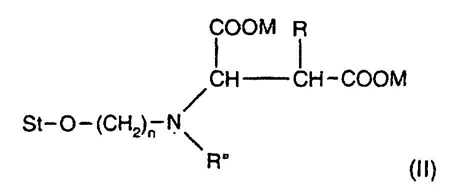 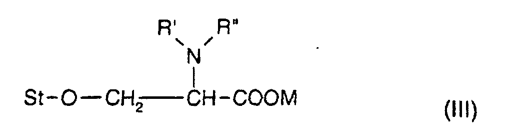 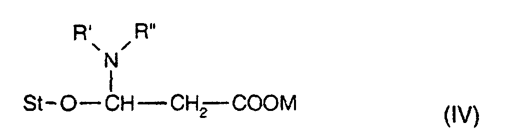 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K 1/2, Li, NH₄, un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460 qui sont inclus à titre de référence.

Les molécules d'amidons peuvent être issues de toutes les sources végétales d'amidon telles que notamment le maïs, la pomme de terre, l'avoine, le riz, le tapioca, le sorgho, l'orge ou le blé. On peut également utiliser les hydrolysats des amidons cités ci-dessus. L'amidon est de préférence issu de la pomme de terre.

On utilise particulièrement les amidons de formules (I) ou (II). On utilise plus particulièrement les amidons modifiés par de l'acide 2-chloroéthyl aminodipropionique, c'est à dire les amidons de formule (I) ou (II) dans lesquelles R, R', R" représentent un atome d'hydrogène et n est égal à 2.

Les amidons amphotères selon l'invention peuvent être utilisés dans les compositions conformes à l'invention dans des concentrations généralement comprises entre 0,01 et 10 %, et de préférence entre 0,1 et 5 % en poids par rapport au poids total de la composition.

Selon un mode de réalisation préféré, la composition comprend en outre au moins un polymère cationique.

De manière encore plus générale, au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) les homopolymères ou copolymères dérivés d'esters ou d'amides acryliques ou méthacryliques et comportant au moins un des motifs de formules suivantes: 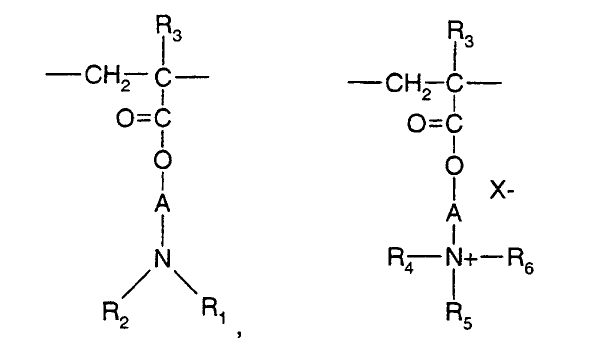 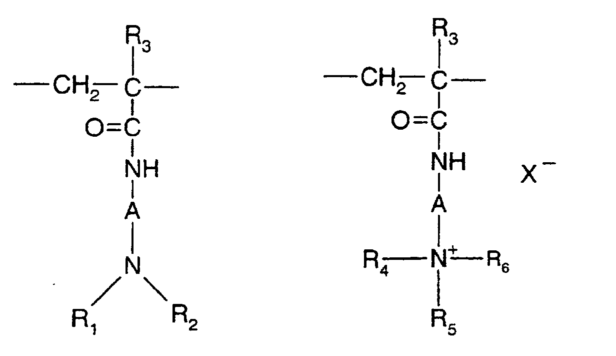 dans lesquelles:
R₃ , identiques ou différents, désignent un atome d'hydrogène ou un radical CH₃;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
R₁ et R₂ , identiques ou différents, représentent hydrogène ou un groupe alkyle ayant de 1 à 6 atomes de carbone et de préférence méthyle ou éthyle;
X désigne un anion dérivé d'un acide minéral ou organique tel que un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.

Ainsi, parmi ces copolymères de la famille (1), on peut citer :
- les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP.
- et les copolymères vinylpyrrolidone / méthacrylamide de diméthylaminopropyle quaternisé tel que le produit vendu sous la dénomination "GAFQUAT HS 100" par la société ISP.

(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.

(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.

Les produits commercialisés répondant à cette définition sont plus particulièrement les produits vendus sous la dénomination "Celquat L 200" et "Celquat H 100" par la Société National Starch.

(4) Les polysaccharides cationiques décrits plus particulièrement dans les brevets US 3 589 578 et 4 031 307 tels que les gommes de guar contenant des groupements cationiques trialkylammonium. On utilise par exemple des gommes de guar modifiées par un sel (par ex. chlorure) de 2,3-époxypropyl triméthylammonium.

De tels produits sont commercialisés notamment sous les dénominations commerciales de JAGUAR C13 S, JAGUAR C 15, JAGUAR C 17 ou JAGUAR C162 par la société MEYHALL.

(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;

(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.

Des polymères de ce type sont en particulier commercialisés sous la dénomination "Hercosett 57" par la société Hercules Inc. ou bien sous la dénomination de "PD 170" ou "Delsette 101 " par la société Hercules dans le cas du copolymère d'acide adipique/époxypropyl/diéthylène-triamine.

(9) les cyclopolymères d'alkyl diallyl amine ou de dialkyl diallyl ammonium tels que les homopolymères ou copolymères comportant comme constituant principal de la chaîne des motifs répondant aux formules (Va) ou (Vb) : 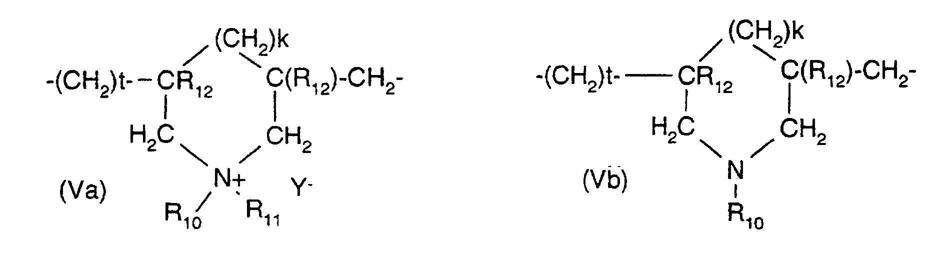 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
R₁₀ et R₁₁, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "Merquat 100" par la société Calgon (et ses homologues de faibles masses moléculaires moyenne en poids) et les copolymères de chlorure de diallyldiméthylammonium et d'acrylamide commercialisés sous la dénomination "MERQUAT 550".

(10) le polymère de diammonium quaternaire contenant des motifs récurrents répondant à la formule (VI) : 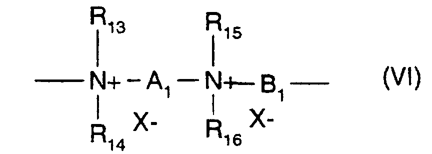 formule (VI) dans laquelle :
R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :

   - (CH2-CH2-O)x-CH2-CH2

   - [CH2-CH(CH3)-O]y-CH2-CH(CH3)-

   où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

   - CH2-CH2-S-S-CH2-CH2- ;
d) un groupement uréylène de formule : -NH-CO-NH- ; n varie de 1 à 6

De préférence, X est un anion tel que le chlorure ou le bromure.

Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule : 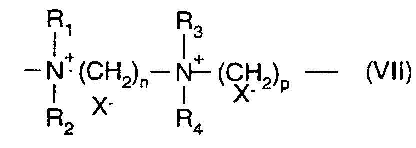 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Un composé de formule (VII) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).

(11) les polymères de polyammonium quaternaires constitués de motifs de formule (VIII): 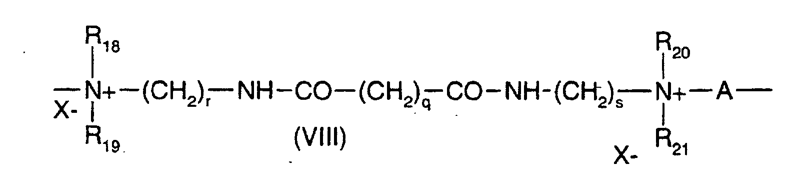 formule dans laquelle :
R₁₈, R₁₉, R₂₀ et R₂₁, identiques ou différents, représentent un atome d'hydrogène ou un radical méthyle, éthyle, propyle, β-hydroxyéthyle, β-hydroxypropyle ou - CH₂CH₂(OCH₂CH₂)ₚOH,
où p est égal à 0 ou à un nombre entier compris entre 1 et 6, sous réserve que R₁₈, R₁₉, R₂₀ et R₂₁ ne représentent pas simultanément un atome d'hydrogène,
r et s, identiques ou différents, sont des nombres entiers compris entre 1 et 6,
q est égal à 0 ou à un nombre entier compris entre 1 et 34,
X⁻ désigne un anion tel qu'un halogènure,
A désigne un radical d'un dihalogénure ou représente de préférence - CH₂-CH₂-O-CH₂-CH₂-.

De tels composés sont notamment décrits dans la demande de brevet EP-A-122 324.

On peut par exemple citer parmi ceux-ci, les produits "Mirapol® A 15", "Mirapol® AD1", "Mirapol® AZ1" et "Mirapol® 175" vendus par la société Miranol.

(12) Les polymères quaternaires de vinylpyrrolidone et de vinylimidazole tels que par exemple les produits commercialisés sous les dénominations Luviquat® FC 905, FC 550 et FC 370 par la société B.A.S.F.

(13) Les polyamines comme le Polyquart® H vendu par HENKEL, référencé sous le nom de « POLYETHYLENEGLYCOL (15) TALLOW POLYAMINE » dans le dictionnaire CTFA.

(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société ALLIED COLLOIDS.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société CALGON, les polysaccharides cationiques tels que les gommes de guar modifiées par un sel de 2,3-époxypropyl triméthylammonium, les polymères quaternaires de vinylpyrrolidone et de vinylimidazole et leurs mélanges.

Selon l'invention, le ou les polymères cationiques peuvent représenter de 0,001 % à 10 % en poids, de préférence de 0,005 % à 5% en poids et plus particulièrement de 0,01 à 3% en poids par rapport au poids total de la composition finale.

Selon un mode de réalisation particulierèment préféré, les compositions selon l'invention comprennent en outre au moins une silicone.

Les silicones utilisables conformément à l'invention peuvent être solubles ou insolubles dans la composition et en particulier être des polyorganosiloxanes insolubles dans la composition ; elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.

On peut également citer les cyclocopolymères du type diméthylsiloxanes méthylakylsiloxane, tel que la "SILICONE VOLATILE FZ 3109" commercialisée par la société UNION CARBIDE, de structure chimique :

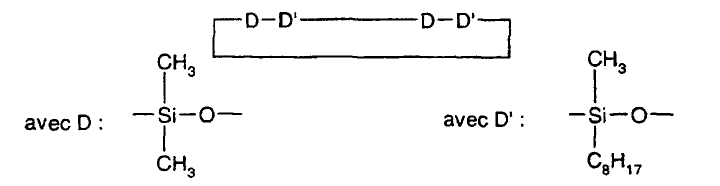

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHODIA telles que par exemple l'huile 70 047 V 500 000 ;
- les huiles de la série MIRASIL commercialisées par la société RHODIA ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA .

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
. les huiles SILBIONE de la série 70 641 de RHODIA ;
. les huiles des séries RHODORSIL 70 633 et 763 de RHODIA ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
. les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 .
- des groupements acyloxyalkyle tels que par exemple les polyorganosiloxanes décrits dans le brevet US-A-4957732.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate; 2-hydroxyalkylthiosulfate tels que les produits commercialisés par la société GOLDSCHMIDT sous les dénominations "ABIL S201 " et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 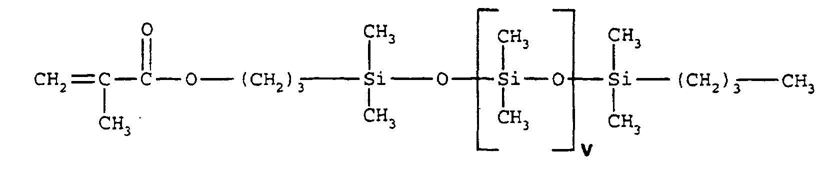 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions, de nanoémulsions ou de micrémulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHODIA, les polyalkylsiloxanes à groupements terminaux diméthylsilanol tels que les diméthiconol ou les polyalkylarylsiloxanes tels que l'huile SILBIONE 70641 V 200 commercialisée par la société RHODIA ;
- la résine d'organopolysiloxane commercialisée sous la dénomination DOW CORNING 593 ;
- les polysiloxanes à groupements aminés tels que les amodiméthicones ou les triméthylsilylamodiméthicone ;

Selon l'invention, le ou les silicones peuvent représenter de 0,001 % à 20 % en poids, de préférence de 0,01 % à 10% en poids et plus particulièrement de 0,1 à 3% en poids par rapport au poids total de la composition finale.

Le milieu aqueux cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.

Les compositions détergentes selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants (associatifs ou non associatifs). On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir de préférence jusqu'à 5 % d'agents nacrants ou opacifiants bien connus dans l'état de la technique tels que par exemple les alcools gras supérieurs à C16, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol.

Les compositions conformes à l'invention peuvent éventuellement contenir en outre au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les agents tensioactifs cationiques, les polymères anioniques ou non ioniques ou amphotères, les protéines, les hydrolysats de protéines, les céramides, les pseudocéramides, les acides gras à chaînes linéaires ou ramifiées en C₁₂-C₄₀ tels que l'acide méthyl-18 eicosanoique, les hydroxyacides, les vitamines, les provitamines tels que le panthénol, les huiles végétales, animales, minérales ou de synthèse et tout autre additif classiquement utilisé dans le domaine cosmétique qui n'affecte pas les propriétés des compositions selon l'invention.

Bien entendu, l'homme du métier veillera à choisir ce ou ces éventuels composés complémentaires et/ou leurs quantités de manière telle que les propriétés avantageuses attachées intrinsèquement à l'association (base lavante + amidon amphotère selon l'invention) conforme à l'invention ne soient pas, ou substantiellement pas, altérées par la ou les adjonctions envisagées.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Ces compositions peuvent se présenter sous la forme de liquides plus ou moins épaissis, de crèmes ou de gels et elles conviennent principalement au lavage, au soin des matières kératiniques en particulier des cheveux et de la peau et encore plus particulièrement des cheveux.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces pour les laver, cette application étant suivie d'un rinçage à l'eau.

Les compositions conformes à l'invention sont également utilisables comme gels douche pour le lavage et le conditionnement des cheveux et/ou de la peau, auquel cas ils sont appliqués sur la peau et/ou les cheveux humides et sont rincés après application.

Des exemples concrets illustrant l'invention vont maintenant être donnés.

### EXEMPLE 1

On a réalisé une composition de shampooing conforme à l'invention (composition A):

| Composition | Invention A |
|---|---|
| Lauryléthersulfate de sodium (C12/C14 à 70/30) à 2.2 moles d'oxyde d'éthylène en solution aqueuse à 28% de MA | 15 g MA |
| Fécule de pomme de terre modifiée par l'acide 2-chloroéthyl aminodipropionique neutralisée par la soude, (Structure Solanace de NATIONAL STARCH) | 0.5 g |
| Hydroxyéthylcellulose réticulée par l'épichlorhydrine et quaternisée par la triméthylamine (JR 400 par la société UNION CARBIDE) | 0.4 g |
| Hydroxyéthylcellulose | 0.4 g |
| Acide citrique q.s. | pH 7 |
| Eau déminéralisée q.s.p. | 100 g |

On effectue un shampooing en appliquant environ 12 g de la composition A sur des cheveux préalablement mouillés. On fait mousser le shampooing puis on rince abondamment à l'eau.

Des essais ont montré que la composition selon l'invention apporte, sur cheveux mouillés un décollement de racines important et beaucoup de légèreté.

### EXEMPLES 2 et 3

| | Exemple 2 | Exemple 3 |
|---|---|---|
| Lauryléthersulfate de sodium (C12/C14 à 70/30) à 2.2 moles d'oxyde d'éthylène | 16 g MA | 16 g MA |
| Cocoyl amidopropyl bétaïne en solution aqueuse à 38% M.A. | 2 g MA | 2 g MA |
| Hydroxyéthylcellulose réticulée à l'épichlorhydrine, quaternisée par la triméthylamine ( Polymer JR 400 de la société UNION CARBIDE) | 0.4 g | ― |
| Chlorure d'hydroxypropyl guar triméthyl ammonium, vendu sous la dénomination Jaguar C13S par la société RHODIA | ― | 0.1 g |
| Fécule de pomme de terre modifiée par l'acide 2-chloroéthyl aminodipropionique neutralisée par la soude (Structure Solanace de NATIONAL STARCH) | 0.2 g | 0.3 g |
| Polydiméthylsiloxane de viscosité 300 000 cSt | - | 2.7 g |
| Polydiméthylsiloxane de viscosité 500 000 cSt | 1.5 g | - |
| Mélange 1-(hexadécyloxy) 2-octadécanol / Alcool cétylique | 3 g | 3 g |
| Monoisopropanolamide de coprah | 0.5 g | 0.5 g |
| Conservateurs, parfum | q.s. | q.s. |
| Agent de pH q.s. | pH 7 | pH 7 |
| Eau déminéralisée q.s.p. | 100 g | 100 g |

On effectue un shampooing en appliquant environ 12 g de la composition sur des cheveux préalablement mouillés. On fait mousser le shampooing puis on rince abondamment à l'eau.

Les cheveux traités avec la composition de l'exemple 2 ou de l'exemple 3 sont doux, légers et se démêlent facilement.

### EXEMPLE 4

On a réalisé un shampooing conforme à l'invention de composition suivante :

| | |
|---|---|
| Lauryléthersulfate de sodium (C12/C14 à 70/30) à 2.2 moles d'oxyde d'éthylène | 15,5 g MA |
| Cocoyl bétaïne en solution aqueuse à 32% M.A. | 3 g MA |
| Chlorure d'hydroxypropyl guar triméthyl ammonium, vendu sous la dénomination Jaguar C13S par la société RHODIA | 0.1 g |
| Fécule de pomme de terre modifiée par l'acide 2-chloroéthyl aminodipropionique neutralisée par la | 0.3 g |
| soude (Structure Solanace de NATIONAL STARCH) Polydiméthylsiloxane de viscosité 60 000 cSt | 2.7 g |
| Amodiméthicone en émulsion cationique à 35% de MA (DC939 de DOW CORNING) | 1,05 gMA |
| Mélange 1-(hexadécyloxy) 2-octadécanol / Alcool cétylique | 2,5 g |
| Monoisopropanolamide de coprah | 0.5 g |
| Conservateurs, parfum | q.s. |
| Acide citrique q.s. | pH 5,5 |
| Eau déminéralisée q.s.p. | 100 g |

On effectue un shampooing en appliquant environ 12 g de la composition sur des cheveux préalablement mouillés. On fait mousser le shampooing puis on rince abondamment à l'eau.
Les cheveux traités avec cette composition sont doux, légers et se démêlent facilement.

## Revendications

1. Composition cosmétique détergente et conditionnante, **caractérisée par le fait qu'**elle comprend, dans un milieu aqueux cosmétiquement acceptable:
4 à 50% en poids par rapport au poids total de la composition d'une base lavante comprenant un ou plusieurs tensioactifs détergents choisis parmi les tensioactifs anioniques, amphotères, non ioniques et leurs mélanges,
et au moins un amidon amphotère choisi parmi les composés de formules (I) à (IV) : 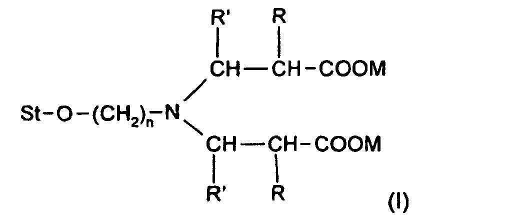 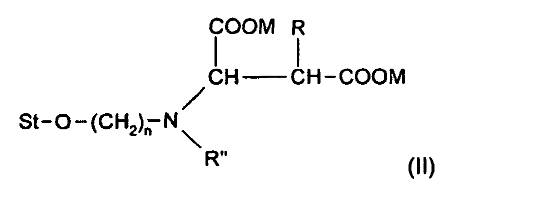 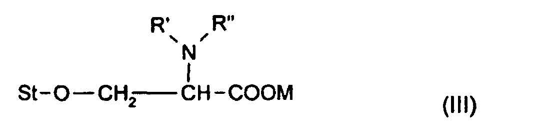 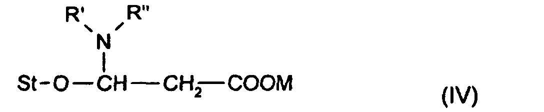 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux, NH₄ , un ammonium quaternaire ou une amine organique,
R" représente un atome d'hydrogène ou un radical alkyle ayant de 1 à 18 atomes de carbone,
la concentration en sels d'un métal alcalin ou alcalinoterreux ou d'une amine grasse et d'un acide gras en C10-C18 étant inférieure ou égale à 1% en poids dans la composition,
à l'exclusion de la composition de shampooing ci-dessous :
| INGREDIENTS | NOM INCI | |
|---|---|---|
| Standapol A | Ammonium lauryl sulfate | 7,50 |
| Standapol EA-3 | Ammonium laureth sulfate | 17,50 |
| Tegobetaine L-7 | Cocamidopropyl betaine | 5,0 |
| Monamid 1113 | Coamide DEA | 3,0 |
| Solance®¹ | Modified Starch | 0,26 |
| Tween 20 | Polysorbate 20 | 1,00 |
| Chlorure de sodium | Sodium Chloride | 0,75 |
| Glydant | DMDM hydantoin | 0,20 |
| Acide citrique | Citric acid | q.s. pH 6 |
| Eau, D.I | Water | q.s. 100 |
| q.s. signifie quantité suffisante | | |
| | | |
|---|---|---|
| ¹ Amidon modifié amphotère de National Starch décrit dans le brevet US 5,871,756. | | |

2. Composition cosmétique détergente et conditionnante, **caractérisée par le fait qu'**elle comprend, dans un milieu aqueux cosmétiquement acceptable:
- 4 à 50% en poids par rapport au poids total de la composition d'une base lavante comprenant un ou plusieurs tensioactifs détergents choisis parmi les tensioactifs anioniques, amphotères, non ioniques et leurs mélanges,
- au moins un polymère cationique et
- au moins un amidon amphotère choisi parmi les composés de formules (I) à (IV) : 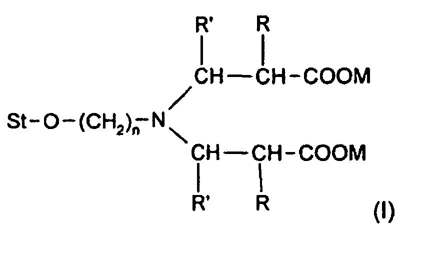 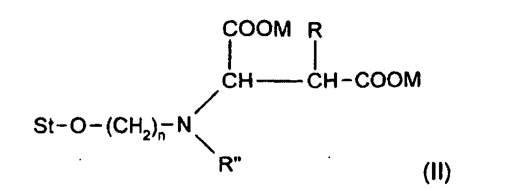 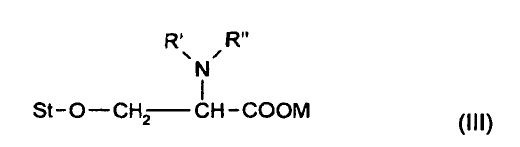 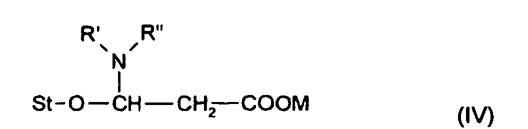
formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux, NH₄ , un ammonium quaternaire ou une amine organique,
R" représente un atome d'hydrogène ou un radical alkyle ayant de 1 à 18 atomes de carbone,
la concentration en sels d'un métal alcalin ou alcalinoterreux ou d'une amine grasse et d'un acide gras en C10-C18 étant inférieure ou égale à 1% en poids dans la composition,

3. Composition selon l'une quelconque des revendications 1 ou 2, **caractérisée par le fait que** les amidons sont de formules (I) ou (II).

4. Composition selon l'une quelconque des revendications 1 à 3, **caractérisée par le fait que** R, R', R" représentent un atome d'hydrogène et n est égal à 2.

5. Composition selon l'une quelconque des revendications 1 à 4, **caractérisée par le fait que** ladite base lavante comprend au moins un ou plusieurs tensioactifs anioniques.

6. Composition selon l'une quelconque des revendications 1 à 5, **caractérisée par le fait que** ladite base lavante est présente à une teneur pondérale comprise entre 6 % et 35 % en poids par rapport au poids total de la composition, de préférence comprise entre 8 % et 25 % en poids.

7. Composition selon l'une quelconque des revendications 1 à 6, **caractérisée par le fait que** l'amidon amphotère est présent dans des concentrations comprises entre 0,01 et 10 %, et de préférence entre 0,1 et 5 % en poids par rapport au poids total de la composition.

8. Composition selon l'une quelconque des revendications 1 et 3 à 7, **caractérisée par le fait qu'**elle comprend en outre au moins un polymère cationique.

9. Composition selon l'une quelconque des revendications 2 à 8, **caractérisée par le fait que** ledit polymère cationique est choisi parmi les dérivés d'éther de cellulose quaternaires, les cyclopolymères cationiques, les polysaccharides cationiques, les polymères quaternaires de vinylpyrrolidone et de vinylimidazole et leurs mélanges.

10. Composition selon la revendication 9, **caractérisée par le fait que** ledit cyclopolymère est choisi parmi les homopolymères du chlorure de diallyldiméthylammonium et les copolymères du chlorure de diallyldiméthylammonium et d'acrylamide.

11. Composition selon la revendication 9, **caractérisée par le fait que** lesdits dérivés d'éther de cellulose quaternaires sont choisis parmi les hydroxyéthylcelluloses ayant réagi avec un époxyde substitué par un groupement triméthylammonium.

12. Composition selon la revendication 9, **caractérisée par le fait que** lesdits polysaccharides cationiques sont choisis parmi les gommes de guar modifiées par un sel de 2,3-époxypropyl triméthylammonium.

13. Composition selon l'une quelconque des revendications 2 à 12 **caractérisée en ce que** le polymère cationique représente de 0,001 % à 10 % en poids, de préférence de 0,005 % à 5 % en poids, et encore plus préférentiellement de 0,01 % à 3 % en poids, du poids total de la composition.

14. Composition selon l'une quelconque des revendications 1 à 13, **caractérisée par le fait qu'**elle comprend en outre au moins une silicone.

15. Composition selon la revendication 14, **caractérisée par le fait que** les silicones sont des polyorganosiloxanes non volatils choisis parmi les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

16. Composition selon la revendication 15, **caractérisée par le fait que** :
(a) les polyalkylsiloxanes sont choisis parmi :
- les polydiméthylsiloxanes à groupements terminaux triméthylsilyle ;
- les polydiméthylsiloxanes à groupements terminaux diméthylsilanol ;
- les polyalkyl(C₁-C₂₀)siloxanes ;
(b) les polyalkylarylsiloxanes sont choisis parmi :
- les polydiméthylméthylphénylsiloxanes, les polydiméthyldiphényl siloxanes linéaires et/ou ramifiés de viscosité comprise entre 1.10⁻⁵ et 5.10⁻²m²/s à 25°C ;
(c) les gommes de silicone sont choisies parmi les polydiorganosiloxanes ayant des masses moléculaires moyennes en nombre comprises entre 200 000 et 1 000 000 utilisés seuls ou sous forme de mélange dans un solvant ;
(d) les résines sont choisies parmi les résines constituées d'unités : R₃ Si O_{1/2}, R₂ Si O_{2/2}, R Si O_{3/2}, Si O_{4/2}
dans lesquelles R représente un groupement hydrocarboné ayant de 1 à 16 atomes de carbone ou un groupement phényle ;
(e) les silicones organo modifiées sont choisies parmi les silicones comportant dans leur structure un ou plusieurs groupements organofonctionnels fixés par l'intermédiaire d'un radical hydrocarboné.

17. Composition selon l'une quelconque des revendications 14 à 16, **caractérisée par le fait que** les silicones sont choisies parmi les polyalkylsiloxanes à groupements terminaux triméthylsilyle, les polyalkylsiloxanes à groupements terminaux diméthylsilanol, les polyalkylarylsiloxanes, les mélanges de deux PDMS constitués d'une gomme et d'une huile de viscosités différentes, les mélanges d'organosiloxanes et de silicones cycliques, les résines d'organopolysiloxanes.

18. Composition selon l'une quelconque des revendications 14 à 17, **caractérisée en ce que** la silicone est présente à une concentration comprise entre 0,001 % et 20 % en poids par rapport au poids total de la composition, de préférence entre 0,01 % et 10 % en poids.

19. Composition selon l'une quelconque des revendications 1 à 18, **caractérisée par le fait qu'**elle comprend en outre au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les agents tensioactifs cationiques, les polymères anioniques ou non ioniques ou amphotères, les protéines, les hydrolysats de protéines, les céramides, les pseudocéramides, les acides gras à chaînes linéaires ou ramifiées en C₁₂-C₄₀ tels que l'acide méthyl-18 eicosanoique, les hydroxyacides, les vitamines, les provitamines tels que le panthénol, les huiles végétales, animales, minérales ou de synthèse et tout autre additif classiquement utilisé dans le domaine cosmétique.

20. Utilisation d'une composition telle définie dans l'une quelconque des revendications 1 à 19 pour le nettoyage et/ou le démaquillage et/ou le conditionnement des matières kératiniques.

21. Utilisation d'une composition telle définie dans l'une quelconque des revendications 1 à 19 comme shampooing des matières kératiniques.

22. Procédé de lavage et de conditionnement des matières kératiniques telles que les cheveux consistant à appliquer sur lesdites matières mouillées une quantité efficace d'une composition telle que définie dans l'une quelconque des revendications 1 à 19, puis à effectuer un rinçage à l'eau après un éventuel temps de pause.

## Claims

1. Detergent and conditioning cosmetic composition, **characterized in that** it comprises, in a cosmetically acceptable aqueous medium:
4 to 50% by weight, relative to the total weight of the composition, of a washing base comprising one or more detergent surfactants chosen from anionic, amphoteric and nonionic surfactants, and mixtures thereof,
and at least one amphoteric starch chosen from the compounds of formulae (I) to (IV): 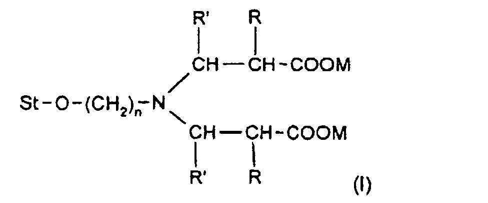 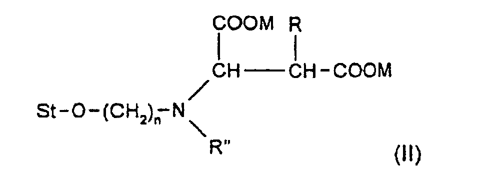 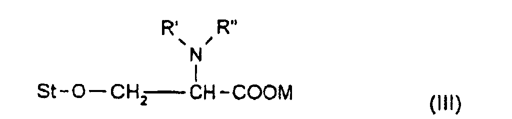 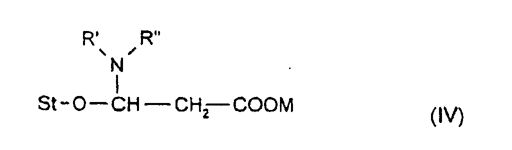 in which formulae:
St-O represents a starch molecule,
R, which may be identical or different, represents a hydrogen atom or a methyl radical,
R', which may be identical or different, represents a hydrogen atom, a methyl radical or a -COOH group,
n is an integer equal to 2 or 3,
M, which may be identical or different, denotes a hydrogen atom, an alkali metal or alkaline-earth metal, NH₄, a quaternary ammonium or an organic amine,
R" represents a hydrogen atom or an alkyl radical containing from 1 to 18 carbon atoms,
the concentration of salts of an alkali metal or alkaline-earth metal or of a fatty amine and of a C10-C18 fatty acid being less than or equal to 1% by weight in the composition,
with the exception of the shampoo composition below:
| INGREDIENTS | INCI NAME | |
|---|---|---|
| Standapol A | Ammonium lauryl sulphate | 7.50 |
| Standapol EA-3 | Ammonium laureth sulphate | 17.50 |
| Tegobetaine L-7 | Cocamidopropyl betaine | 5.0 |
| Monamid 1113 | Coamide DEA | 3.0 |
| Solance®¹ | Modified starch | 0.26 |
| Tween 20 | Polysorbate 20 | 1.00 |
| Sodium chloride | Sodium chloride | 0.75 |
| Glidant | DMDM hydantoin | 0.20 |
| Citric acid | Citric acid | q.s. pH 6 |
| Water, D.I. | Water | q.s. 100 |
| q.s. signifies quantum sufficit | | |
| | | |
|---|---|---|
| ¹ Amphoteric modified starch from National Starch, described in US patent 5,871,756. | | |

2. Detergent and conditioning cosmetic composition, **characterized in that** it comprises, in a cosmetically acceptable aqueous medium:
- 4 to 50% by weight, relative to the total weight of the composition, of a washing base, comprising one or more detergent surfactants chosen from anionic, amphoteric and nonionic surfactants, and mixtures thereof,
- at least one cationic polymer and
- at least one amphoteric starch chosen from the compounds of formulae (I) to (IV): 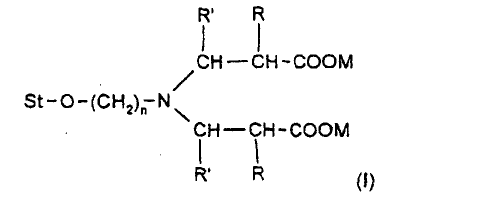 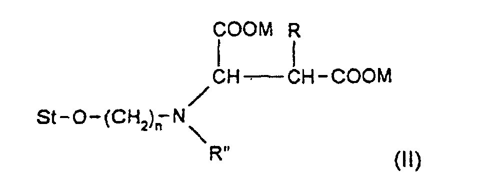 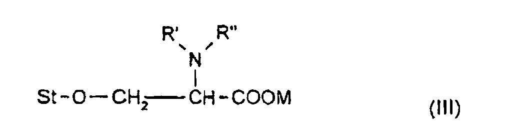 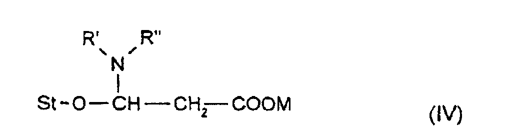
in which formulae:
St-O represents a starch molecule,
R, which may be identical or different, represents a hydrogen atom or a methyl radical,
R', which may be identical or different, represents a hydrogen atom, a methyl radical or a -COOH group,
n is an integer equal to 2 or 3,
M, which may be identical or different, denotes a hydrogen atom, an alkali metal or alkaline-earth metal, NH₄, a quaternary ammonium or an organic amine,
R" represents a hydrogen atom or an alkyl radical containing from 1 to 18 carbon atoms,
the concentration of salts of an alkali metal or alkaline-earth metal or of a fatty amine and of a C10-C18 fatty acid being less than or equal to 1% by weight in the composition.

3. Composition according to either of Claims 1 and 2, **characterized in that** the starches are of formula (I) or (II).

4. Composition according to any one of Claims 1 to 3, **characterized in that** R, R' and R" represent a hydrogen atom and n is equal to 2.

5. Composition according to any one of Claims 1 to 4, **characterized in that** the said washing base comprises at least one or more anionic surfactants.

6. Composition according to any one of Claims 1 to 5, **characterized in that** the said washing base is present at a weight content of between 6% and 35% by weight relative to the total weight of the composition, preferably between 8% and 25% by weight.

7. Composition according to any one of Claims 1 to 6, **characterized in that** the amphoteric starch is present in concentrations of between 0.01% and 10% and preferably between 0.1% and 5% by weight relative to the total weight of the composition.

8. Composition according to any one of Claims 1 and 3 to 7, **characterized in that** it also comprises at least one cationic polymer.

9. Composition according to any one of Claims 2 to 8, **characterized in that** the said cationic polymer is chosen from quaternary cellulose ether derivatives, cationic cyclopolymers, cationic polysaccharides, quaternary polymers of vinylpyrrolidone and of vinylimidazole, and mixtures thereof.

10. Composition according to Claim 9, **characterized in that** the said cyclopolymer is chosen from diallyldimethylammonium chloride homopolymers and copolymers of diallyldimethylammonium chloride and of acrylamide.

11. Composition according to Claim 9, **characterized in that** the said quaternary cellulose ether derivatives are chosen from hydroxyethylcelluloses which have reacted with an epoxide substituted with a trimethylammonium group.

12. Composition according to Claim 9, **characterized in that** the said cationic polysaccharides are chosen from guar gums modified with a 2,3-epoxypropyltrimethylammonium salt.

13. Composition according to any one of Claims 2 to 12, **characterized in that** the cationic polymer represents from 0.001% to 10% by weight, preferably from 0.005% to 5% by weight and even more preferably from 0.01% to 3% by weight, relative to the total weight of the composition.

14. Composition according to any one of Claims 1 to 13, **characterized in that** it also comprises at least one silicone.

15. Composition according to Claim 14, **characterized in that** the silicones are non-volatile polyorganosiloxanes chosen from polyalkylsiloxanes, polyarylsiloxanes, polyalkylarylsiloxanes, silicone gums and resins, and polyorganosiloxanes modified with organofunctional groups, as well as mixtures thereof.

16. Composition according to Claim 15, **characterized in that**
(a) the polyalkylsiloxanes are chosen from:
- polydimethylsiloxanes containing trimethylsilyl end groups;
- polydimethylsiloxanes containing dimethylsilanol end groups;
- poly(C₁-C₂₀)alkylsiloxanes;
(b) the polyalkylarylsiloxanes are chosen from:
- polydimethylmethylphenylsiloxanes and polydimethyldiphenylsiloxanes which are linear and/or branched, with a viscosity of between 1 × 10⁻⁵ and 5 × 10⁻² m²/s at 25°C;
(c) the silicone gums are chosen from polydiorganosiloxanes with number-average molecular masses of between 200 000 and 1 000 000, used alone or in the form of a mixture in a solvent;
(d) the resins are chosen from resins consisting of units: R₃SiO_{1/2}, R₂SiO_{2/2}, RSiO_{3/2}, SiO_{4/2}
in which R represents a hydrocarbon-based group containing from 1 to 16 carbon atoms or a phenyl group;
(e) the organomodified silicones are chosen from silicones comprising, in their structure, one or more organofunctional groups attached via a hydrocarbon-based radical.

17. Composition according to any one of Claims 14 to 16, **characterized in that** the silicones are chosen from polyalkylsiloxanes containing trimethylsilyl end groups, polyalkylsiloxanes containing dimethylsilanol end groups, polyalkylarylsiloxanes, mixtures of two PDMSs consisting of a gum and an oil of different viscosities, mixtures of organosiloxanes and of cyclic silicones, and polyorganosiloxane resins.

18. Composition according to any one of Claims 14 to 17, **characterized in that** the silicone is present in a concentration of between 0.001% and 20% by weight relative to the total weight of the composition, preferably between 0.01% and 10% by weight.

19. Composition according to any one of Claims 1 to 18, **characterized in that** it also comprises at least one additive chosen from foam synergists such as C₁₀-C₁₈ 1,2-alkanediols or fatty alkanolamides derived from monoethanolamine or from diethanolamine, silicone or non-silicone sunscreens, cationic surfactants, anionic or nonionic or amphoteric polymers, proteins, protein hydrolysates, ceramides, pseudoceramides, fatty acids containing linear or branched C₁₂-C₄₀ chains such as 18-methyleicosanoic acid, hydroxy acids, vitamins, provitamins such as panthenol, plant, animal, mineral or synthetic oils and any other additive conventionally used in the cosmetics field.

20. Use of a composition as defined in any one of Claims 1 to 19 for cleaning and/or removing make-up from and/or conditioning keratin materials.

21. Use of a composition as defined in any one of Claims 1 to 19 as a shampoo for keratin materials.

22. Process for washing and conditioning keratin materials such as the hair, which consists in applying to the said wet materials an effective amount of a composition as defined in any one of Claims 1 to 19, and then in rinsing with water after an optional time of pause.

## Patentansprüche

1. Reinigende und konditionierende kosmetische Zusammensetzung, **dadurch gekennzeichnet, dass** sie in einem kosmetisch akzeptablen wässerigen Medium enthält:
4 bis 50 Gew.-%, bezogen auf das Gesamtgewicht der Zusammensetzung, einer Waschbasis, die einen oder mehrere reinigende grenzflächenaktive Stoffe enthält, die unter den anionischen, amphoteren und nichtionischen grenzflächenaktiven Stoffen sowie ihren Gemischen ausgewählt sind,
und mindestens eine amphotere Stärke, die ausgewählt ist unter den Verbindungen der Formeln (I) bis (IV): 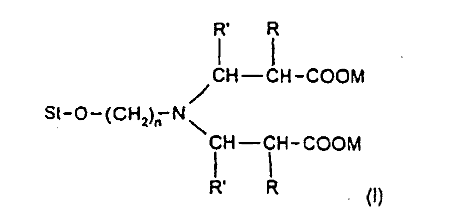 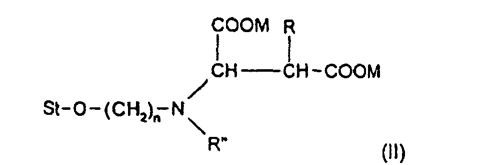 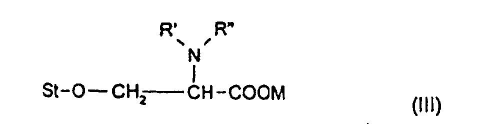 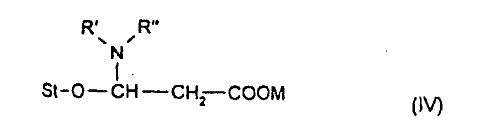 worin bedeuten:
St-O ein Stärkemolekül,
R, gleich oder verschieden, ein Wasserstoffatom oder eine Methylgruppe,
R', gleich oder verschieden, ein Wasserstoffatom, eine Methylgruppe oder eine Gruppe -COOH,
n eine ganze Zahl gleich 2 oder 3,
M, gleich oder verschieden, ein Wasserstoffatom, ein
Alkalimetall oder ein Erdalkalimetall, NH₄, ein quaternäres Ammonium oder ein organisches Amin,
R" ein Wasserstoffatom oder eine Alkylgruppe mit 1 bis 18 Kohlenstoffatomen,
wobei die Konzentration an Salzen eines Alkalimetalls oder Erdalkalimetalls oder eines Fettamins mit einer C₁₀₋₁₈-Fettsäure in der Zusammensetzung kleiner als oder gleich 1 Gew.-% ist,
wobei die nachstehende Shampoo-Zusammensetzung ausgenommen ist:
| BESTANDTEIL | INCI-BEZEICHNUNG | |
|---|---|---|
| Standapol A | Ammonium lauryl sulfate | 7,50 |
| Standapol EA-3 | Ammonium laureth sulfate | 17,50 |
| Tegobetain L-7 | Cocamidopropyl betaine | 5,0 |
| Monamid 1113 | Coamide DEA | 3,0 |
| Solance®¹ | Modified Starch | 0,26 |
| Tween 20 | Polysorbate 20 | 1,00 |
| Natriumchlorid | Sodium Chloride | 0,75 |
| Glydant | DMDM hydantoin | 0,20 |
| Citronensäure | Citric acid | q.s. pH 6 |
| Wasser, entionisiert | Water | q.s. 100 |
| q.s. (quantum satis) bedeutet ausreichende Menge | | |
| | | |
|---|---|---|
| ¹ Modifizierte amphotere Stärke von National Starch, beschrieben in dem Patent US 5 871 756. | | |

2. Reinigende und konditionierende kosmetische Zusammensetzung; **dadurch gekennzeichnet, dass** sie in einem kosmetisch akzeptablen wässerigen Medium enthält:
- 4 bis 50 Gew.-%, bezogen auf das Gesamtgewicht der Zusammensetzung, einer Waschbasis, die einen oder mehrere reinigende grenzflächenaktive Stoffe enthält, die unter anionischen, amphoteren und nichtionischen grenzflächenaktiven Stoffen sowie ihren Gemischen ausgewählt sind,
- mindestens ein kationisches Polymer und
- mindestens eine amphotere Stärke, die ausgewählt ist unter den Verbindungen der Formeln (I) bis (IV): 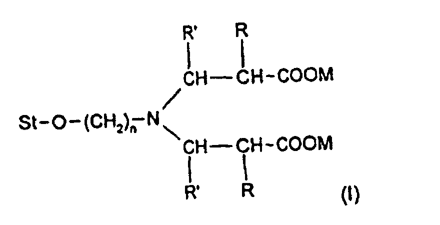 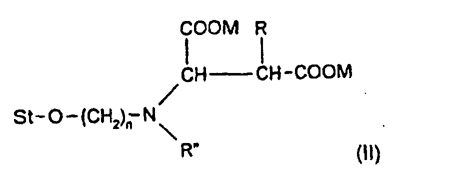 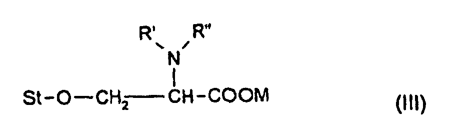 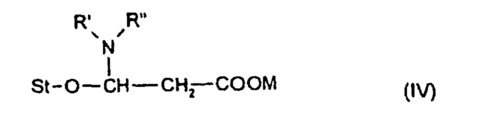
worin bedeuten:
St-O ein Stärkemolekül,
R, gleich oder verschieden, ein Wasserstoffatom oder eine Methylgruppe,
R', gleich oder verschieden, ein Wasserstoffatom, eine Methylgruppe oder eine Gruppe -COOH,
n eine ganze Zahl gleich 2 oder 3,
M, gleich oder verschieden, ein Wasserstoffatom, ein Alkalimetall oder ein Erdalkalimetall, NH₄, ein quaternäres Ammonium oder ein organisches Amin,
R" ein Wasserstoffatom oder eine Alkylgruppe mit 1 bis 18 Kohlenstoffatomen,
wobei die Konzentration an Salzen eines Alkalimetalls oder Erdalkalimetalls oder eines Fettamins mit einer C₁₀₋₁₈-Fettsäure in der Zusammensetzung kleiner als oder gleich 1 Gew.-% ist.

3. Zusammensetzung nach Anspruch 1 oder 2, **dadurch gekennzeichnet, dass** die Stärken die Formel (I) oder die Formel (II) besitzen.

4. Zusammensetzung nach einem der Ansprüche 1 bis 3, **dadurch gekennzeichnet, dass** R, R', R" ein Wasserstoffatom bedeuten und n gleich 2 ist.

5. Zusammensetzung nach einem der Ansprüche 1 bis 4, **dadurch gekennzeichnet, dass** die Waschbasis mindestens einen oder mehrere anionische grenzflächenaktive Stoffe enthält.

6. Zusammensetzung nach einem der Ansprüche 1 bis 5, **dadurch gekennzeichnet, dass** die Waschbasis in einem Gewichtsanteil von 6 bis 35 Gew.-%, bezogen auf das Gesamtgewicht der Zusammensetzung, und vorzugsweise in einem Gewichtsanteil von 8 bis 25 Gew.-% vorliegt.

7. Zusammensetzung nach einem der Ansprüche 1 bis 6, **dadurch gekennzeichnet, dass** die amphotere Stärke in Konzentrationen von 0,01 bis 10 Gew.-% und vorzugsweise 0,1 bis 5 Gew.-%, bezogen auf das Gesamtgewicht der Zusammensetzung, vorliegt.

8. Zusammensetzung nach einem der Ansprüche 1 und 3 bis 7, **dadurch gekennzeichnet, dass** sie ferner mindestens ein kationisches Polymer enthält.

9. Zusammensetzung nach einem der Ansprüche 2 bis 8, **dadurch gekennzeichnet, dass** das kationische Polymer ausgewählt ist unter quaternären Celluloseetherderivaten, kationischen Cyclopolymeren, kationischen Polysacchariden, quaternären Polymeren von Vinylpyrrolidon und Vinylimidazol sowie ihren Gemischen.

10. Zusammensetzung nach Anspruch 9, **dadurch gekennzeichnet, dass** das Cyclopolymer ausgewählt ist unter Homopolymeren von Diallyldimethylammoniumchlorid und Copolymeren von Diallyldimethylammoniumchlorid und Acrylamid.

11. Zusammensetzung nach Anspruch 9, **dadurch gekennzeichnet, dass** die quaternären Celluloseetherderivate ausgewählt sind unter Hydroxyethylcellulosen, die mit einem mit einer Trimethylammoniumgruppe substituierten Epoxid umgesetzt wurden.

12. Zusammensetzung nach Anspruch 9, **dadurch gekennzeichnet, dass** die kationischen Polysaccharide ausgewählt sind unter Guargummen, die mit einem 2,3-Epoxypropyltrimethylammoniumsalz modifiziert wurden.

13. Zusammensetzung nach einem der Ansprüche 2 bis 12, **dadurch gekennzeichnet, dass** das kationische Polymer 0,001 bis 10 Gew.-%, vorzugsweise 0,005 bis 5 Gew.-% und noch bevorzugter 0,01 bis 3 Gew.-% des Gesamtgewichts der Zusammensetzung ausmacht.

14. Zusammensetzung nach einem der Ansprüche 1 bis 13, **dadurch gekennzeichnet, dass** sie ferner mindestens ein Silicon enthält.

15. Zusammensetzung nach Anspruch 14, **dadurch gekennzeichnet, dass** die Silicone nichtflüchtige Polyorganosiloxane sind, die ausgewählt sind unter Polyalkylsiloxanen, Polyarylsiloxanen, Polyalkylarylsiloxanen, Siliconkautschuken und Siliconharzen und mit organofunktionellen Gruppen modifizierten Polyorganosiloxanen sowie ihren Gemischen.

16. Zusammensetzung nach Anspruch 15, **dadurch gekennzeichnet, dass**
(a) die Polyalkylsiloxane ausgewählt sind unter
- Polydimethylsiloxanen mit Trimethylsilyl-Endgruppen;
- Polydimethylsiloxanen mit Dimethylsilanol-Endgruppen;
- Polyalkyl(C₁₋₂₀)siloxanen;
(b) die Polyalkylarylsiloxane ausgewählt sind unter
- geradkettigen und/oder verzweigten Polydimethylmethylphenylsiloxanen und Polydimethyldiphenylsiloxanen einer Viskosität von 1·10⁵ bis 5·10² m²/s bei 25 °C;
(c) die Siliconkautschuke ausgewählt sind unter Polydiorganosiloxanen mit Zahlenmitteln der Molekülmasse im Bereich von 200000 bis 1000000 und allein oder in Form von Gemischen mit einem Lösungsmittel verwendet werden;
(d) die Harze ausgewählt sind unter Harzen, die bestehen aus Einheiten R₃SiO_{1/2}, R₂SiO_{2/2}, RSiO_{3/2}, SiO_{4/2}, wobei R eine Kohlenwasserstoffgruppe mit 1 bis 16 Kohlenstoffatomen oder eine Phenylgruppe bedeutet;
(e) die organomodifizierten Silicone ausgewählt sind unter Siliconen, die in ihrer Struktur eine oder mehrere organofunktionelle Gruppen enthalten, die über eine Kohlenwasserstoffgruppe gebunden sind.

17. Zusammensetzung nach einem der Ansprüche 14 bis 16, **dadurch gekennzeichnet, dass** die Silicone ausgewählt sind unter Polyalkylsiloxanen mit Trimethylsilyl-Endgruppen, Polyalkylsiloxanen mit Dimethylsilanol-Endgruppen, Polyalkylarylsiloxanen, Gemischen von 2 PDMS, die aus einem Kautschuk und einem Öl bestehen, die unterschiedliche Viskosität besitzen, Gemischen von Organosiloxanen und cyclischen Siliconen und Organopolysiloxanharzen.

18. Zusammensetzung nach einem der Ansprüche 14 bis 17, **dadurch gekennzeichnet, dass** das Silicon in einer Konzentration von 0,001 bis 20 Gew.-%, bezogen auf das Gesamtgewicht der Zusammensetzung, und vorzugsweise von 0,01 bis 10 Gew.-% vorliegt.

19. Zusammensetzung nach einem der Ansprüche 1 bis 18, **dadurch gekennzeichnet, dass** sie ferner mindestens ein Additiv enthält, das ausgewählt ist unter Schaumsynergisten wie C₁₀₋₁₈-Alkandiolen oder von Mono- oder Diethanolamin abgeleiteten Alkanolamiden, gegebenenfalls Silicon enthaltenden Sonnenfiltern, kationischen grenzflächenaktiven Mitteln, anionischen oder nichtionischen oder amphoteren Polymeren, Proteinen, Proteinhydrolysaten, Ceramiden, Pseudoceramiden, geradkettigen oder verzweigten C₁₂₋₄₀-Fettsäuren, wie 18-Methyleicosansäure, Hydroxysäuren, Vitaminen, Provitaminen wie Panthenol, Pflanzenölen, tierischen Ölen, Mineralölen oder synthetischen Ölen sowie beliebigen anderen Additiven, die herkömmlicherweise auf dem Gebiet der Kosmetik verwendet werden.

20. Verwendung einer Zusammensetzung wie in einem der Ansprüche 1 bis 19 definiert zur Reinigung und/oder zum Abnehmen von Schminke und/oder zur Konditionierung von Keratinmaterialien.

21. Verwendung einer Zusammensetzung wie in einem der Ansprüche 1 bis 19 definiert als Shampoo für Keratinmaterialien.

22. Verfahren zum Waschen und zur Konditionierung von Keratinmaterialien wie Haaren, das darin besteht, auf die feuchten Keratinmaterialien eine wirksame Menge einer Zusammensetzung wie in einem der Ansprüche 1 bis 19 definiert aufzubringen und dann nach einer eventuellen Wartezeit ein Spülen mit Wasser vorzunehmen.
